# FASCICULE DE BREVET EUROPEEN

(11) **EP 3 038 690 B1**
(45) Date de publication et mention de la délivrance du brevet: **15.07.2020**
(21) Numéro de dépôt: 14777258.6
(22) Date de dépôt: 02.09.2014
(51) Int. Cl.: A61M 25/00, A61F 5/44

(54) **POCHE DESTINÉE À RECUEILLIR DES URINES**
BEUTEL ZUM SAMMELN VON URIN
BAG FOR COLLECTING URINE

(30) Priorité: 02.07.2013 FR 1301561
(43) Date de publication de la demande: 06.07.2016
(73) Titulaire: B. Braun Medical SAS, 92210 Saint-Cloud (FR)
(72) Inventeur: BLOCQUEL, Laura, 28402 Nogent-le-Rotrou (FR); PREZELIN, Anthony, 28400 Nogent-le-Rotrou (FR)
(74) Mandataire: August Debouzy
(86) Numéro de dépôt international: PCT/EP2014/002376
(87) Numéro de publication internationale: WO 2015/000602

(56) Documents cités:
- EP-A1- 2 072 075
- DE-A1- 2 227 416
- DE-A1-102007 018 275
- FR-A1- 2 942 714
- US-A- 2 856 932
- US-A- 3 762 399
- US-A1- 2008 119 803

## Description

L'invention concerne une poche destinée à recueillir des urines.

Les poches urinaires sont utilisées pour recueillir l'urine lorsqu'un patient est cathétérisé. L'urine peut alors couler depuis la vessie à travers le cathéter dans la poche urinaire. Pour cela, un cathéter peut être posé durablement, par exemple, lorsque le patient doit garder le lit et que l'urine doit être déviée durablement. Un cathéter peut être également posé à court terme, uniquement pour vider une fois la vessie. Dans ce dernier cas, on parle de cathétérisation intermittente qui est par exemple utilisée lorsqu'un patient est paraplégique et que la vessie doit être vidée régulièrement par cathétérisation ou en cas de problème d'incontinence. Habituellement, la cathétérisation intermittente est réalisée par le patient lui-même plusieurs fois par jour.

Notamment dans le cadre de la cathétérisation intermittente par le patient lui-même, la manipulation confortable de la poche urinaire avant et après l'utilisation est importante.

Le document EP 2 072 075 A1 décrit par exemple une poche urinaire avec un prolongement central contenant un cathéter. Ce prolongement peut être rabattu sur la poche puis les deux bords latéraux de celle-ci, jouxtant chaque côté du prolongement rabattu, peuvent être ensuite repliés par-dessus l'un sur l'autre. La poche peut ainsi être pliée pour pouvoir être rangée facilement et de manière compacte par le patient. Un patient qui pratique l'auto-cathéterisation intermittente a besoin chaque jour de 4 à 6 cathéters. Il doit conséquemment emmener plusieurs poches avec cathéter pour être autonome. Les poches contenant un cathéter sont d'habitude emballées ensembles et le patient doit manipuler cet emballage ou doit emmener des poches en vrac. Les deux possibilités engendrent des inconvénients pour des patients avec mobilité réduite.

L'objet de l'invention est de créer une poche urinaire qui peut être pliée facilement pour être peu encombrant avant son utilisation et qui peut être manipulée facilement par un patient, notamment un patient paraplégique.

Cette tâche est remplie par le set de poche de recueil d'urines et de cathéter urinaire selon la présente invention, défini dans la revendication indépendante 1. Différents modes de réalisation préférentiels sont définis dans les revendications dépendantes.

Ainsi, un set de cathéter urinaire et poche de recueil prêt à l'emploi peut être mis à disposition du patient, en particulier pour la cathétérisation intermittente. Le cathéter urinaire peut être placé dans le prolongement de la poche avec son extrémité distale vers le haut.

La poche prête à l'emploi, non utilisée, avec le cathéter à l'intérieur, peut être alors rangée avantageusement de manière compacte en pliant la poche horizontalement une fois et en rabattant la prolongation vers le bas, puis en l'enroulant. Les cathéters habituels possèdent sur leur extrémité proximale un connecteur plat. L'extrémité de la poche pliée comportant ce connecteur peut alors être roulée dans un rayon plus grand que l'extrémité opposée. Ainsi, cela donne une forme conique de l'ensemble enroulé. Plusieurs poches enroulées de cette manière peuvent être empilées les unes dans les autres. Un patient peut de manière discrète avoir un stock suffisant pour une journée avec lui. Les poches sont bien protégées contre des endommagements, même une fois sorties de leur suremballage, et sont faciles à manipuler.

La poche peut alors posséder sur l'extrémité supérieure du prolongement de préférence une couture prédécoupée permettant de déchirer le prolongement si bien que l'extrémité distale du cathéter peut être glissée à travers le prolongement. L'extrémité proximale du cathéter reste dans le prolongement de la poche. Le connecteur situé habituellement sur la partie proximale du cathéter peut prendre la fonction de butée et empêche le cathéter de sortir entièrement de la poche. Le cathéter peut ensuite être utilisé de la manière habituelle.

Selon l'invention, la hauteur verticale du prolongement représente environ la moitié de la hauteur totale de la poche. La poche peut ainsi être pliée une seule fois avant ou après avoir replié le prolongement avec le cathéter vers la poche, puis être enroulée. L'ensemble peut être déplié facilement par un patient même avec des capacités manuelles limitées.

Avantageusement, la poche de recueil selon l'invention peut être équipée d'une valve anti-retour à l'entrée du compartiment de recueil qui empêche un reflux du fluide en direction de l'entrée.

Selon un mode préféré de l'invention, le pourtour soudé de la partie basse du compartiment de recueil de la poche correspond essentiellement à un secteur de cercle dont l'angle est au moins 180°.

Grace à cette forme particulière, la poche peut se tenir droite une fois remplie et ne bascule pas sur le côté. Le patient peut poser la poche remplie sur une surface plane, par exemple pour se rhabiller, et la poche y reste sans s'écouler.

L'angle du secteur essentiellement circulaire de la partie basse du compartiment de recueil de la poche peut avantageusement être compris entre 180° et 330°, et en particulier entre 225° et 270°. Le compartiment de recueil atteint ainsi sa largeur maximale avant de se rétrécir vers le haut. Des essais ont montré que la stabilité de la poche remplie peut être améliorée de cette façon.

La forme de la partie basse du compartiment peut essentiellement être symétrique selon un axe vertical. La poche se rétrécie ainsi régulièrement des deux cotés.

La poche peut présenter un rétrécissement au niveau de son compartiment et peut s'élargir vers le haut pour former un rétrécissement limité verticalement au niveau du compartiment de recueil.

De préférence, la partie circulaire du compartiment de recueil présente un volume entre 350 et 550 ml et notamment de 450 ml. Des essais ont montrés qu'une poche ayant un compartiment de ces dimensions présente une stabilité à partir d'un contenu d'environs 200 ml de liquide. Le volume d'urine évacué par un patient est d'habitude entre 200 ml et 400 ml, tel qu'une poche selon l'invention remplie de ce volume de liquide reste stable sur une surface plane.

Avantageusement, le compartiment de recueil peut présenter un volume maximal d'environs 750 ml. Ceci correspond au volume maximal d'urine évacué par un patient. Une poche de ces dimensions ne peut donc pas déborder, même si le volume versé est bien supérieur au volume habituellement accueilli.

Un exemple de réalisation de l'invention est expliqué plus en détail dans la suite à l'aide des dessins annexés :
- **Figure 1**: représente un exemple de réalisation d'une poche de recueil intégrant un cathéter urinaire féminin ;
- **Figure 2**: représente l'exemple de réalisation selon la figure 1 dans son état enroulé.

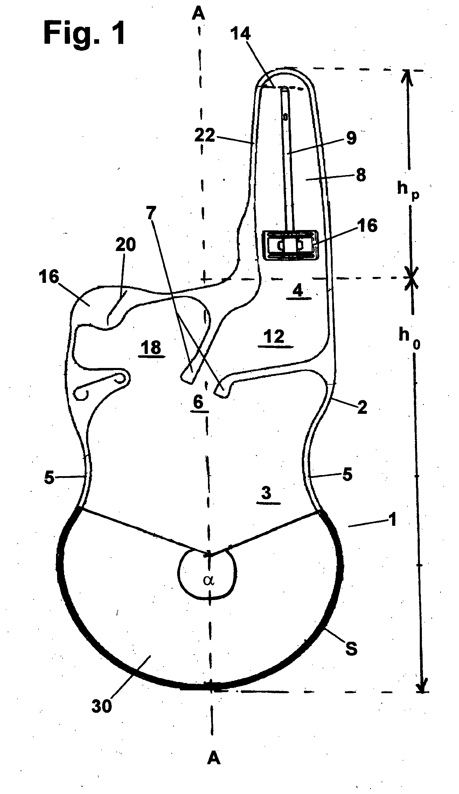

**La** **figure 1** présente un exemple de réalisation privilégié d'une poche **1** selon l'invention destinée à recueillir des urines. Un cathéter urinaire féminin **9** est intégré dans un prolongement **8** de la poche.

La poche **1** est constituée de deux feuilles en matière plastique superposées et soudées le long de leur pourtour **2.**

La poche **1** comprend un compartiment de recueil **3** pour recueillir les urines, un orifice d'admission **4** et une entrée **6** vers la chambre de recueil **3** connectée à l'orifice d'admission **4** par un canal **12.** L'entrée **6** délimite le compartiment **3** vers le haut.

La poche dispose sur son orifice d'admission **4** d'un prolongement vertical **8** qui est soudé le long de son pourtour **22** tel que la poche est totalement fermée. Dans le prolongement **8** est situé un cathéter urinaire féminin **9.**

L'extrémité distale du cathéter **9** peut être glissée ensuite à travers la poche par le déchirement d'une couture **14** prévue à cet effet sur l'extrémité de la prolongation **8** et peut être utilisée de la manière habituelle. L'extrémité proximale du cathéter **9** avec le connecteur **16** reste ainsi à l'intérieur de la prolongation **8** si bien que l'urine peut s'écouler directement dans la chambre **3** de la poche **1.**

La poche **1** dispose également d'une valve anti-retour simple qui est formée par les deux soudures **7** et qui empêche le reflux de liquide par l'entrée **6** dans le canal **12** dès que la chambre a atteint un certain niveau de remplissage.

L'ensemble peut être stérilisée si bien que le cathéter urinaire **9** peut être conditionné prêt à l'emploi.

La poche de recueil **1** présente la hauteur **h₀** qui correspond à peu près au double de la hauteur **hₚ** du prolongement **8.** Le cathéter dispose d'un connecteur **16** plat sur son extrémité proximale.

La poche peut être ainsi rangée de manière compacte en rabattant tout d'abord le prolongement **8** avec le cathéter **9** vers le bas et en pliant la poche **1** à l'horizontale environ à la moitié de sa hauteur. La poche peut être ensuite enroulée.

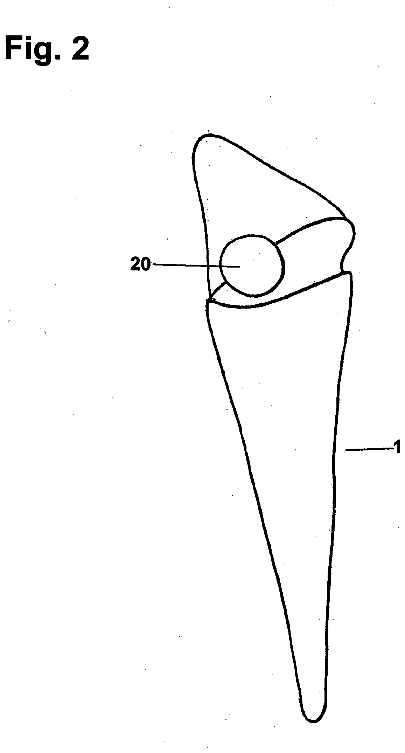

La **figure 2** montre la poche **1** selon la figure 1 une fois enroulée. Comme on peut le voir, un cône est créé par le connecteur du cathéter dans le prolongement rabattu vers l'intérieur. La poche enroulée peut être fixé par un petit autocollant **20.**

Les patients peuvent garder sur eux la poche enroulée **1** avec le cathéter prêt à l'emploi, de manière compacte et discrète. Un patient qui pratique l'auto-cathétérisation intermittente a besoin chaque jour de 4 à 6 cathéters. Étant donné que les poches enroulées en forme de cône peuvent être empilées facilement, les patients peuvent également emmener facilement plusieurs poches avec eux. Les poches enroulées et empilées sont bien protégées contres des endommagements même sans suremballage.

En revenant à la figure 1, on peut voir que le pourtour soudé de la partie basse **30** du compartiment **3** de la poche **1** correspond essentiellement à un secteur de cercle **S** dont l'angle est environ 225°. La forme de la partie basse **30** dudit compartiment **3** est essentiellement symétrique selon un axe vertical **A.** La poche présente un rétrécissement **5** au niveau du compartiment **3.**

Grâce à cette structure de la poche, la poche peut tenir debout sur une surface plane sans basculer à partir d'un niveau de remplissage d'environ 200 ml. La poche peut être déposée après utilisation pendant un instant, afin par exemple que le patient puisse se rhabiller ou pour prélever un échantillon ultérieurement avant de jeter le liquide qu'elle contient.

La partie circulaire du compartiment de recueil **3** présente un volume d'environ 450 ml. Une fois la vessie entièrement vidée, il se trouve normalement entre 200 et 400 ml d'urine dans la chambre **3** de la poche **1.** Le patient peut alors poser la poche remplie un court instant afin de se rhabiller. Grâce à sa structure selon l'invention, la poche **1** reste stable, ne bascule pas et ne se renverse pas.

Le volume maximal du compartiment de recueil **3** est d'environs 750 ml pour éviter tout débordement en cas de gros volume d'urine à accueillir.

La poche **1** comprend en outre une zone **16** qui comprend une petite chambre **18** qui peut être ouverte par une couture de déchirement **20** afin de vider la poche.

## Revendications

1. Set de poche de recueil (1) d'urines et de cathéter urinaire (9), la poche de recueil (1) étant constituée de deux feuilles en matière plastique superposées et soudées le long de leur pourtour (2), ladite poche de recueil (1) comportant :
- un compartiment de recueil· (3) ;
- un orifice d'admission (4) et
- une entrée (6) dudit compartiment de recueil (3) en communication avec l'orifice d'admission (4) délimitant ledit compartiment de recueil (3) vers le haut,
dans lequel
- l'orifice d'admission est muni d'un prolongement vertical (8) fermé le long de son pourtour (22) ledit cathéter urinaire (9) étant intégré dans le prolongement vertical (8) ;
**caractérisé en ce que** :
- l'orifice d'admission (4) et l'entrée (6) sont décalés verticalement; et
- la hauteur verticale (ho) de la poche (1) hors prolongement vertical (8) correspond au double de la hauteur (hp) dudit prolongement (8), de telle sorte que le prolongement vertical (8) avec le cathéter (9) peut être rabattu vers le bas en pliant la poche (1) à l'horizontale environ à la moitié de sa hauteur et la poche peut ensuite être enroulée, permettant ainsi à la poche d'être rangée de manière compacte.

2. Set selon la revendication 1 **caractérisé en ce que** le cathéter (9) présente un connecteur plat (16) à son extrémité proximale.

3. Set selon l'une quelconque des revendications 1 à 2, **caractérisé en ce que** la poche (1) présente une valve anti-retour (7) à l'entrée (6) dudit compartiment (3).

4. Set selon l'une quelconque des revendications 1 à 3, **caractérisé en ce que** le pourtour soudé de la partie basse (30) du compartiment (3) correspond essentiellement à un secteur de cercle (S) dont l'angle (a) est au moins 180°.

5. Set selon la revendication 4, **caractérisé en ce que** l'angle (α) du secteur de cercle (S) de la partie essentiellement circulaire est compris entre 180° et 330°, et en particulier entre 225° et 270°.

6. Set selon l'une quelconque des revendications 4 ou 5, **caractérisée en ce que** la forme de la partie basse (30) dudit compartiment (3) est essentiellement symétrique selon un axe vertical (A).

7. Set selon l'une quelconque des revendications 4 à 6, **caractérisée en ce que** la poche présente un rétrécissement (5) au niveau du compartiment (3).

8. Set selon l'une quelconque des revendications 1 à 7, **caractérisée en ce que** la partie circulaire du compartiment de recueil (3) présente un volume entre 350 et 550 ml et notamment de 450 ml.

9. Set selon l'une quelconque des revendications 1 à 8, **caractérisée en ce que** le compartiment de recueil (3) présente un volume maximal d'environ 750 ml.

## Patentansprüche

1. Set aus einem Beutel zum Sammeln (1) von Urin und Urinkatheter (9), wobei der Sammelbeutel (1) aus zwei Folien aus Kunststoff gebildet ist, die übereinandergelegt und entlang ihres Umfangs (2) verschweißt sind, wobei der Sammelbeutel (1) aufweist:
- ein Sammelfach (3);
- eine Zulauföffnung (4) und
- einen Eingang (6) des Sammelfachs (3) in Kommunikation mit der Zulauföffnung (4), die das Sammelfach (3) nach oben begrenzt,
wobei
- die Zulauföffnung mit einer vertikalen Verlängerung (8) ausgestattet ist, die entlang ihres Umfangs (22) verschlossen ist, wobei der Urinkatheter (9) in die vertikale Verlängerung (8) integriert ist;
**dadurch gekennzeichnet, dass**
- die Zulauföffnung (4) und der Eingang (6) vertikal versetzt sind; und
- die vertikale Höhe (ho) des Beutels (1) ohne vertikale Verlängerung (8) dem Doppelten der Höhe (hₚ) der Verlängerung (8) entspricht, so dass die vertikale Verlängerung (8) mit dem Katheter (9) nach unten durch Falten der Tasche (1) in der Horizontalen etwa auf die Hälfte ihrer Höhe heruntergeklappt werden kann und die Tasche dann gewickelt werden kann, was der Tasche erlaubt, kompakt verstaut zu werden.

2. Set (1) nach Anspruch 1, **dadurch gekennzeichnet, dass** der Katheter (9) einen flachen Verbinder (16) an seinem proximalen Ende aufweist.

3. Set nach einem der Ansprüche 1 bis 2, **dadurch gekennzeichnet, dass** der Beutel (1) ein Rücklaufsperrventil (7) am Eingang (6) des Fachs (3) aufweist.

4. Set (1) nach einem der Ansprüche 1 bis 3, **dadurch gekennzeichnet, dass** der geschweißte Umfang des unteren Abschnitts (30) des Fachs (3) im Wesentlichen einem Kreisbogen (S) entspricht, dessen Winkel (α) mindestens 180° beträgt.

5. Set nach Anspruch 4, **dadurch gekennzeichnet, dass** der Winkel (a) des Kreisbogens (S) des im Wesentlichen kreisrunden Abschnitts zwischen 180° und 330° und insbesondere zwischen 225° und 270° beträgt.

6. Set nach einem der Ansprüche 4 oder 5, **dadurch gekennzeichnet, dass** die Form des unteren Abschnitts (30) des Fachs (3) gemäß einer vertikalen Achse (A) im Wesentlichen symmetrisch ist.

7. Set nach einem der Ansprüche 4 bis 6, **dadurch gekennzeichnet, dass** der Beutel eine Verengung (5) im Bereich des Fachs (3) aufweist.

8. Set nach einem der Ansprüche 1 bis 7, **dadurch gekennzeichnet, dass** der kreisrunde Abschnitt des Sammelfachs (3) ein Volumen zwischen 350 und 550 ml und insbesondere von 450 ml aufweist.

9. Set nach einem der Ansprüche 1 bis 8, **dadurch gekennzeichnet, dass** das Sammelfach (3) ein maximales Volumen von zirka 750 ml aufweist.

## Claims

1. A set of urine collection pouch (1) and urinary catheter (9), the collection pouch (1) being made up of two superimposed sheets of plastic welded along their perimeter (2), said collection pouch (1) including:
- a collection compartment (3);
- an intake orifice (4) and
- an inlet (6) of said collection compartment (3) in communication with the intake orifice (4) upwardly delimiting said collection compartment (3),
wherein
- the intake orifice is provided with a vertical extension (8) closed along its perimeter (22), said urinary catheter (9) being integrated into the vertical extension (8); **characterized in that**:
- the intake orifice (4) and the inlet (6) are vertically offset; and
- the vertical height (ho) of the pouch (1) excluding vertical extension (8) corresponds to twice the height (hp) of said extension (8), such that the vertical extension (8) with the catheter (9) can be folded over in the downward direction by folding the pouch (1) horizontally at approximately half its height and the pouch can next be wound, thus allowing the pouch to be stored in a compact manner.

2. The set according to claim 1, **characterized in that** the catheter (9) has a flat connector (16) at its proximal end.

3. The set according to any one of claims 1 to 2, **characterized in that** the pouch (1) has a nonreturn valve (7) at the inlet (6) of said compartment (3).

4. The set according to any one of claims 1 to 3, **characterized in that** the welded perimeter of the lower part (30) of the compartment (3) essentially corresponds to a circle sector (S), the angle (a) of which is at least 180°.

5. The set according to claim 4, **characterized in that** the angle (a) of the circle sector (S) of the essentially circular part is between 180° and 330°, and in particular between 225° and 270°.

6. The set according to any one of claims 4 or 5, **characterized in that** the shape of the lower part (30) of said compartment (3) is essentially symmetrical along a vertical axis (A).

7. The set according to any one of claims 4 to 6, **characterized in that** the pouch has a narrowing (5) at the compartment (3).

8. The set according to any one of claims 1 to 7, **characterized in that** the circular part of the collection compartment (3) has a volume between 350 and 550 mL, and in particular of 450 mL.

9. The set according to any one of claims 1 to 8, **characterized in that** the collection compartment (3) has a maximum volume of about 750 mL.
